# EUROPEAN PATENT APPLICATION

(11) **EP 4 582 013 A1**
(43) Date of publication of application: **09.07.2025**
(21) Application number: 23859977.3
(22) Date of filing: 07.08.2023
(51) Int. Cl.: A61B 3/10

(54) **OPHTHALMIC DEVICE AND METHOD FOR CONTROLLING OPHTHALMIC DEVICE**

(30) Priority: 02.09.2022 JP 2022140108
(71) Applicant: TOPCON CORPORATION, Tokyo 174-8580 (JP)
(72) Inventor: KATO Yuichi, Tokyo 174-8580 (JP)
(74) Representative: Schmitt-Nilson Schraud Waibel Wohlfrom Patentanwälte Partnerschaft mbB
(86) International application number: PCT/JP2023/028670
(87) International publication number: WO 2024/048209

(57) **Abstract**

There are provided an ophthalmic device and a control method for the ophthalmic device capable of easily producing satisfactory three-dimensional data of a site to be observed of a subject eye. The ophthalmic device includes a pre-scan controlling unit (216) configured to execute a pre-scan on a site to be observed (a fundus (Ef)), a cross-sectional shape acquiring unit (218) configured to detect or predict a cross-sectional shape of the site to be observed, a main scan controlling unit (220) configured to control an optical scanner and execute a main scan on the site to be observed, an optical path length acquiring unit (214) configured to acquire an optimum optical path length at a particular scanning position of the main scan at least before the main scan, an optical path length adjustment controlling unit (224) configured to, during execution of the main scan, control an optical path length changing unit to adjust an optical path length to the optimum optical path length, and a three-dimensional data producing unit (226) configured to produce three-dimensional data (404) of the site to be observed.

## Description

### {Technical Field}

The present invention relates to an ophthalmic device that acquires a tomographic image of a subject eye using optical coherence tomography and a control method for the same.

### {Background Art}

Ophthalmic devices that photograph a tomographic image of a subject eye using optical coherence tomography (OCT) are known. For example, the ophthalmic device according to PTL 1 includes an interference optical system that splits light from a light source into measurement light and reference light, irradiates a subject eye with the measurement light, and detects interference light between return light from the subject eye and the reference light. An optical scanner that performs scanning with measurement light and an optical path length changing unit, such as a corner cube or a reflector, that changes an optical path length of reference light or measurement light are provided in the interference optical system.

The ophthalmic device according to PTL 1 irradiates a fundus (a site to be observed) of a subject eye with measurement light to acquire a one-dimensional signal in a depth direction of the fundus (A-scan) and performs a one-dimensional scan (line scan) on the subject eye with the measurement light to acquire a B-scan image including a fundus tomographic image (B-scan). The ophthalmic device according to PTL 1 also has a function of adjusting an optical path length of light such as reference light to display a predetermined region of the fundus tomographic image at a reference position (Z designated position) in the B-scan image (auto-Z), a function of maintaining the fundus tomographic image at the reference position (Z-lock), and a function of changing the reference position (Z-lock position change processing).

The ophthalmic device according to PTL 2 executes three-dimensional OCT photographing that repeatedly performs a B-scan while changing a B-scan scanning position in a direction perpendicular to both an A-scan direction and a B-scan direction and acquires a B-scan image for each scanning position, thereby producing three-dimensional data of a fundus (C-scan).

In executing such three-dimensional OCT photographing, display positions of fundus tomographic images within respective B-scan images (frames) acquired at a scanning start position, a scanning middle position, and a scanning end position for B-scans the differ due to a curve of an eyeball. If a scanning range of measurement light is narrow, almost the whole of a fundus tomographic image fits within each B-scan image.

Depending on three-dimensional OCT photographing conditions (e.g., a scanning range and a curvature of an eyeball), the whole of a fundus tomographic image may fail to be displayed within a B-scan image to produce a reversed image or a part of the fundus tomographic image may be folded back. The fundus tomographic image may not be normally displayed. To cope with such a case, adjustment of a display position of a fundus tomographic image within a B-scan image as in the ophthalmic device according to PTL 1 is conceivable. However, since a reference position is set low such that the whole of a fundus tomographic image fits within a B-scan image particularly when a scanning range of measurement light is set wide, a position of a fundus tomographic image within a B-scan image is likely to move downward due to movement of an eyeball of a subject eye to produce a reversed image. In this case, a height position (average value) of the tomographic image within the B-scan image may remain low to keep the reference position unchanged. When the scanning range of measurement light is set wide, a sharp fundus tomographic image may not be obtained. This may reduce the accuracy of fundus tomographic image position detection required for auto-Z, Z-lock, and Z-lock position changing processing described above.

For this reason, the ophthalmic device according to PTL 2 acquires, in advance, A-scan images at a plurality of positions in a B-scan scanning direction and adjusts an optical path length of reference light on the basis of the A-scan images at the plurality of positions such that the whole of a tomographic image of a site to be observed is arranged at an optimum position within a B-scan image.

The ophthalmic device according to PTL 3 adjusts an optical path length of at least one of measurement light or reference light in accordance with a scanning range of measurement light at the time of a B-scan such that the whole of a tomographic image of a site to be observed is arranged at an optimum position within a B-scan image.

### {Citation List}

### {Patent Literature}

{PTL 1} Japanese Patent Application Laid-Open No. 2016-41221
{PTL 2} Japanese Patent Application Laid-Open No. 2015-16151
{PTL 3} Japanese Patent Application Laid-Open No. 2020-110256

### {Summary of Invention}

### {Technical Problem}

In three-dimensional OCT photographing of a fundus of a subject eye, a curve of a fundus tomographic image within a B-scan image becomes stronger as a scanning range of measurement light becomes wider. For this reason, the method described in each of the above-described patent literatures may be unable to cope with the problem depending on a B-scan scanning position, i.e., a fundus tomographic image within a B-scan image may deviate from a reference position to prevent normal display of a part of the fundus tomographic image. This may result in a failure to produce satisfactory three-dimensional data of a fundus.

The present invention has been made in view of the above-described circumstances, and has as its object to provide an ophthalmic device and a control method for the ophthalmic device capable of easily producing satisfactory three-dimensional data of a site to be observed of a subject eye.

### {Solution to Problem}

An ophthalmic device for achieving the object of the present invention includes an interference optical system configured to split light emitted from a light source into measurement light and reference light, irradiate a site to be observed of a subject eye with the measurement light, and detect interference light between the reference light and return light from the site to be observed irradiated with the measurement light, an optical path length changing unit provided in the interference optical system and configured to change an optical path length of at least one of the measurement light and the reference light, an optical scanner configured to scan the site to be observed with the measurement light, a pre-scan controlling unit configured to control the optical scanner and execute a pre-scan that is a B-scan on the site to be observed, a cross-sectional shape acquiring unit configured to form a B-scan image of the site to be observed on the basis of a detection signal of the interference light detected by the interference optical system during execution of the pre-scan and detect or predict a cross-sectional shape of the site to be observed on the basis of the B-scan image, a main scan controlling unit configured to control the optical scanner and execute, as a main scan, a three-dimensional scan that repeatedly executes the B-scan while changing a scanning position of the B-scan on the site to be observed, an optical path length acquiring unit configured to, if the optical path length that allows adjustment of a position of a tomographic image of the site to be observed within the B-scan image to a reference position set in advance is an optimum optical path length, acquire the optimum optical path length at a particular scanning position of the main scan at least before execution of the main scan, an optical path length adjustment controlling unit configured to, during execution of the main scan, control the optical path length changing unit to adjust the optical path length to the optimum optical path length on the basis of the cross-sectional shape detected or predicted by the cross-sectional shape acquiring unit and the optimum optical path length acquired by the optical path length acquiring unit, and a three-dimensional data producing unit configured to produce three-dimensional data of the site to be observed on the basis of the detection signal of the interference light detected by the interference optical system during execution of the main scan.

According to the ophthalmic device, it is possible to adjust an image of the site to be observed within a B-scan image formed at each scanning position of the main scan to a reference position by adjusting the optical path length to the optimum optical path length during execution of the main scan.

In the ophthalmic device according to another aspect of the present invention, if a direction in which the B-scan on the site to be observed is to be performed, the direction perpendicular to both an A-scan direction and a B-scan direction, is a perpendicular direction, the main scan controlling unit controls the optical scanner, and repeatedly executes, as the main scan, the B-scan while changing the scanning position along the perpendicular direction.

In the ophthalmic device according to another aspect of the present invention, the pre-scan controlling unit controls the optical scanner and executes, as the pre-scan, the B-scan in the perpendicular direction on the site to be observed, and the cross-sectional shape acquiring unit detects a particular layer of the site to be observed from the B-scan image and detects the cross-sectional shape on the basis of a shape of the particular layer. This allows high-accuracy detection of the cross-sectional shape of the site to be observed.

In the ophthalmic device according to another aspect of the present invention, the pre-scan controlling unit controls the optical scanner and executes, as the pre-scan, the B-scan at a plurality of the scanning positions different from each other, and the cross-sectional shape acquiring unit forms a plurality of the B-scan images corresponding one-to-one with the scanning positions of the pre-scan and predicts the cross-sectional shape on the basis of a result of detecting the position of the tomographic image within the B-scan image for each of the scanning positions. Since the cross-sectional shape of the site to be observed can be obtained in a short time, three-dimensional OCT photographing can be executed in a short time.

In the ophthalmic device according to another aspect of the present invention, an operation mode of the main scan by the main scan controlling unit is selectively switchable between normal mode in which a scanning range of the measurement light is set at a first range and wide-angle mode in which the scanning range is set at a second range wider than the first range, and the pre-scan controlling unit, the cross-sectional shape acquiring unit, the optical path length acquiring unit, and the optical path length adjustment controlling unit are actuated only when the operation mode is the wide-angle mode. With this configuration, if the operation mode of the main scan is the normal mode, three-dimensional OCT photographing can be executed in a short time by skipping the pre-scan.

The ophthalmic device according to another aspect of the present invention includes a display device and a live display controlling unit configured to execute, before the pre-scan, the B-scan at the particular scanning position by the optical scanner, and formation of the B-scan image based on the detection signal of the interference light detected by the interference optical system and display of the B-scan image by the display device, the live display controlling unit adjusts the optical path length to the optimum optical path length on the basis of the detection signal of the interference light detected by the interference optical system, and the optical path length acquiring unit acquires the optimum optical path length adjusted by the live display controlling unit before the pre-scan.

The ophthalmic device according to another aspect of the present invention includes an image forming unit configured to form the respective B-scan images for the scanning positions on the basis of respective detection signals of the interference light detected by the interference optical system at the scanning positions during execution of the main scan, and the three-dimensional data producing unit includes a three-dimensional data producing unit configured to produce the three-dimensional data on the basis of the respective B-scan images formed for the scanning positions by the image forming unit.

A control method for an ophthalmic device for achieving the object of the present invention is a control method for an ophthalmic device including an interference optical system configured to split light emitted from a light source into measurement light and reference light, irradiate a site to be observed of a subject eye with the measurement light, and detect interference light between the reference light and return light from the site to be observed irradiated with the measurement light, an optical path length changing unit provided in the interference optical system and configured to change an optical path length of at least one of the measurement light and the reference light, and an optical scanner configured to scan the site to be observed with the measurement light, the method including a cross-sectional shape acquiring step of forming a B-scan image of the site to be observed on the basis of a detection signal of the interference light detected by the interference optical system during execution of a pre-scan that is a B-scan on the site to be observed by the optical scanner and detecting or predicting a cross-sectional shape of the site to be observed on the basis of the B-scan image, an optical path length acquiring step of, if a three-dimensional scan that repeatedly executes the B-scan while changing a scanning position of the B-scan on the site to be observed is a main scan, and the optical path length that allows adjustment of a position of a tomographic image of the site to be observed within the B-scan image to a reference position set in advance is an optimum optical path length, acquiring the optimum optical path length at a particular scanning position of the main scan at least before execution of the main scan, an optical path length adjustment controlling step of,during execution of the main scan, controlling the optical path length changing unit to adjust the optical path length to the optimum optical path length on the basis of the cross-sectional shape detected or predicted in the cross-sectional shape acquiring step and the optimum optical path length acquired in the optical path length acquiring step, and a three-dimensional data producing step of producing three-dimensional data of the site to be observed on the basis of the detection signal of the interference light detected by the interference optical system during execution of the main scan.

### {Advantageous Effects of Invention}

The present invention can easily produce satisfactory three-dimensional data of a site to be observed of a subject eye.

### {Brief Description of Drawings}

Figure 1 is a schematic diagram showing an example of a configuration of an ophthalmic device according to a first embodiment;
Figure 2 is a schematic diagram of an optical system of an OCT unit;
Figure 3 is a functional block diagram of a calculation controlling unit of the first embodiment;
Figure 4 is an explanatory chart for explaining three-dimensional OCT photographing;
Figure 5 is an explanatory chart for explaining scanning with measurement light LS from before three-dimensional OCT photographing [3D (H) scan] to completion of the photographing;
Figure 6 is an explanatory chart for explaining scanning with the measurement light LS from before three-dimensional OCT photographing [3D (V) scan] to completion of the photographing;
Figure 7 is an explanatory chart for explaining adjustment of a target optical path length by an optical path length adjustment controlling unit during execution of a main scan;
Figure 8 is a flowchart showing a flow of a three-dimensional OCT photographing process on a fundus by the ophthalmic device of the first embodiment;
Figure 9 is an explanatory chart comparing B-scan images at scanning positions of a main scan to be formed by a conventional method and B-scan images at scanning positions of a main scan to be formed by the ophthalmic device 1 of the first embodiment;
Figure 10 is an explanatory chart for explaining a pre-scan according to a second embodiment;
Figure 11 is an explanatory graph for explaining a fundus cross-sectional shape prediction process by a cross-sectional shape acquiring unit of the second embodiment;
Figure 12 is a flowchart showing a flow of a three-dimensional OCT photographing process on a fundus by an ophthalmic device of the second embodiment;
Figure 13 is a functional block diagram of a calculation controlling unit of an ophthalmic device according to a third embodiment; and
Figure 14 is a flowchart showing a flow of a three-dimensional OCT photographing process on a fundus by the ophthalmic device of the third embodiment.

### {Description of Embodiments}

### [Ophthalmic Device According to First Embodiment]

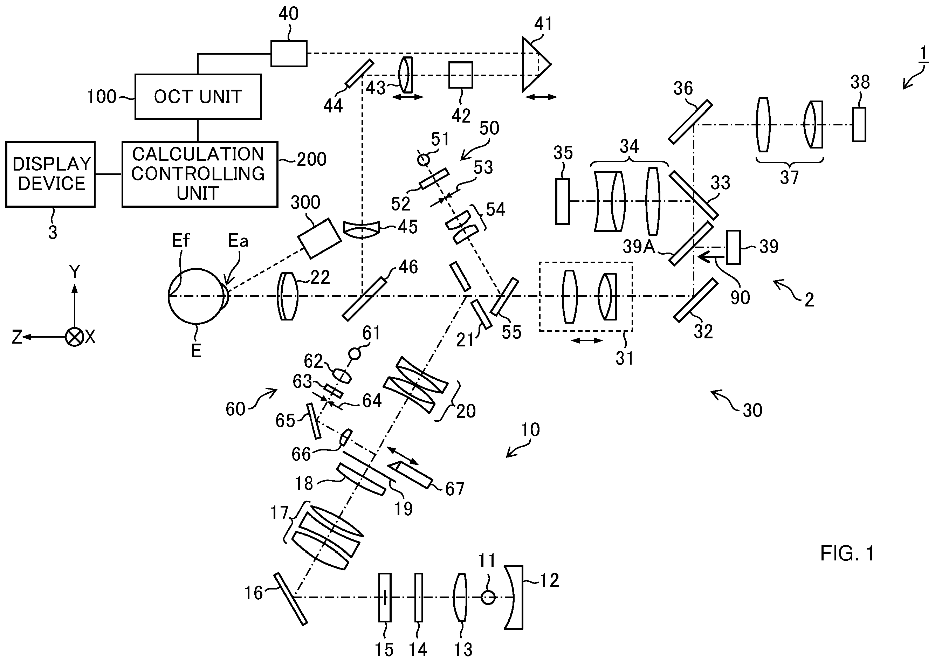

Figure 1 is a schematic diagram showing an example of a configuration of an ophthalmic device 1 according to a first embodiment. The ophthalmic device 1 is a multifunction machine that is a combination of a fundus camera that photographs a fundus Ef as a site to be observed of a subject eye E and an optical coherence tomograph that executes OCT photographing (OCT measurement or OCT scanning) that obtains a fundus tomographic image of the fundus Ef using OCT. Note that a Z direction in Figure 1 is a front-back direction in which the ophthalmic device 1 moves toward or away from the subject eye E, a Y direction is an up-down direction, and that an X direction is a left-right direction.

As shown in Figure 1, the ophthalmic device 1 includes a fundus camera unit 2, an OCT unit 100, a calculation controlling unit 200, and one pair of anterior eye cameras 300. The fundus camera unit 2 has almost the same optical system as that of a conventional fundus camera. The OCT unit 100 has an optical system for executing OCT photographing of the fundus Ef. The calculation controlling unit 200 is a calculation processing unit, such as a personal computer, that executes various types of calculation processing and control processing.

### [Fundus Camera Unit]

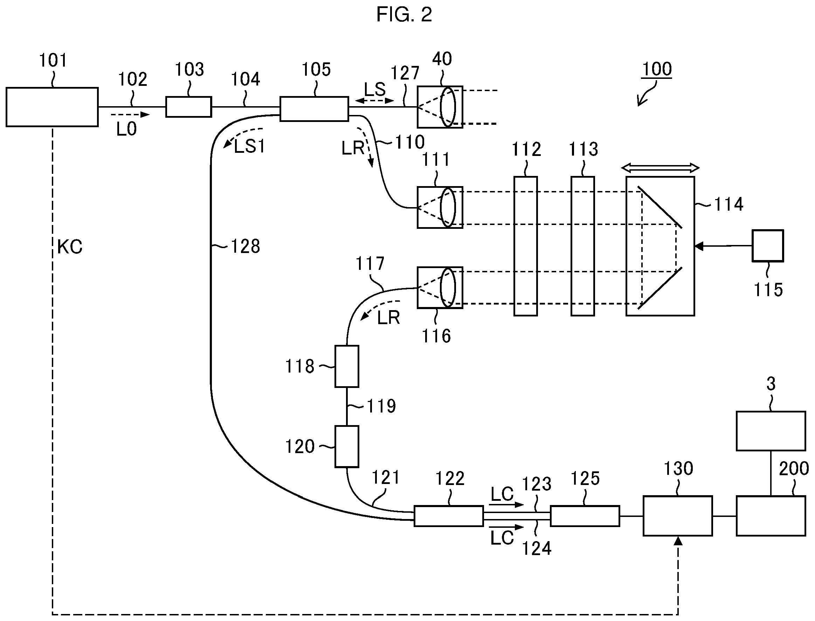

The fundus camera unit 2 includes an illumination optical system 10 and a photographic optical system 30 as an optical system for acquiring a two-dimensional image (fundus image) representing a surface conformation of the fundus Ef of the subject eye E. The illumination optical system 10 illuminates the fundus Ef with illumination light. The photographic optical system 30 guides fundus-reflected light reflected from the fundus Ef of the illumination light to CMOS (Complementary Metal Oxide Semiconductor) type or CCD (Charge Coupled Device) type image sensors 35 and 38. The photographic optical system 30 also guides measurement light LS (see Figure 2) output from the OCT unit 100 to the fundus Ef and guides return light LS1 (see Figure 2) returned from the fundus Ef to the OCT unit 100.

The illumination optical system 10 includes an observation light source 11, a reflection mirror 12, a condenser lens 13, a visible light cut filter 14, a photographing light source 15, a mirror 16, relay lenses 17 and 18, a diaphragm 19, a relay lens 20, a perforated mirror 21, a dichroic mirror 46, and an objective lens 22.

The photographic optical system 30 includes a dichroic mirror 55, a focal lens 31, a mirror 32, a half mirror 39A, a target displaying unit 39, a dichroic mirror 33, a condenser lens 34, the image sensor 35, a mirror 36, a condenser lens 37, and the image sensor 38 in addition to the objective lens 22, the dichroic mirror 46, and the perforated mirror 21.

As the observation light source 11, for example, a halogen lamp or an LED (Light Emitting Diode) light source is used to emit observation illumination light. Observation illumination light emitted from the observation light source 11 is reflected by the reflection mirror 12, travels through the condenser lens 13, and penetrates the visible light cut filter 14 to become near-infrared light. Further, the observation illumination light is converged near the photographing light source 15, is reflected by the mirror 16, and travels through the relay lenses 17 and 18, the diaphragm 19, and the relay lens 20. The observation illumination light is reflected by a peripheral portion (a region around an aperture) of the perforated mirror 21, penetrates the dichroic mirror 46, and is refracted by the objective lens 22 to illuminate the fundus Ef.

Fundus-reflected light of the observation illumination light is refracted by the objective lens 22, penetrates the dichroic mirror 46, passes through the aperture formed in a center region of the perforated mirror 21, penetrates the dichroic mirror 55, travels through the focal lens 31, and is reflected by the mirror 32. The fundus-reflected light penetrates the half mirror 39A, is reflected by the dichroic mirror 33, and is caused by the condenser lens 34 to form an image on a light-receiving surface of the image sensor 35. The image sensor 35 subjects the fundus-reflected light to image pickup (receives the light) and outputs an image pickup signal. An observation image based on the image pickup signal output from the image sensor 35 is displayed on a display device 3. Note that an observation image of an anterior eye part Ea of the subject eye E is displayed on the display device 3 when the focus of the photographic optical system 30 is adjusted to the anterior eye part Ea and that the observation image of the fundus Ef is displayed on the display device 3 when the focus of the photographic optical system 30 is adjusted to the fundus Ef.

As the photographing light source 15, for example, a xenon lamp or an LED light source is used to emit photographing illumination light. Photographing illumination light emitted from the photographing light source 15 is applied to the fundus Ef through the same route as that of the observation illumination light described earlier. Fundus-reflected light of the photographing illumination light is guided to the dichroic mirror 33 through the same route as that of the fundus-reflected light of the observation illumination light, penetrates the dichroic mirror 33, is reflected by the mirror 36, and is caused by the condenser lens 37 to form an image on a light-receiving surface of the image sensor 38.

The image sensor 38 subjects the fundus-reflected light to image pickup (receives the light) and outputs an image pickup signal. A photographed image based on the image pickup signal output from the image sensor 38 is displayed on the display device 3. Note that the display device 3 that displays an observation image and the display device 3 that displays a photographed image may be the same device or different devices.

As the target displaying unit 39, for example, a dot-matrix liquid crystal display (LCD) and a matrix light-emitting diode (LED) are used. The target displaying unit 39 displays a fixation target 90. Since the target displaying unit 39 is a dot-matrix LCD, for example, a display form (e.g., a shape) and a display position of the fixation target 90 can be arbitrarily set.

Part of a pencil from the fixation target 90 displayed on the target displaying unit 39 is reflected by the half mirror 39A and is then projected onto the subject eye E via the mirror 32, the focal lens 31, the dichroic mirror 55, the aperture of the perforated mirror 21, the dichroic mirror 46, and the objective lens 22. This allows presentation of the fixation target 90 to the subject eye E.

The fundus camera unit 2 further includes an alignment optical system 50 and a focus optical system 60 like a conventional fundus camera. The alignment optical system 50 produces an alignment indicator for registration (alignment) of the fundus camera unit 2 with respect to the subject eye E. The focus optical system 60 produces a split indicator for bringing the fundus Ef into focus.

The alignment optical system 50 includes an LED 51, diaphragms 52 and 53, and a relay lens 54 in addition to the objective lens 22, the dichroic mirror 46, the perforated mirror 21, and the dichroic mirror 55. The focus optical system 60 includes an LED 61, a relay lens 62, a split indicator plate 63, a two-aperture diaphragm 64, a mirror 65, a condenser lens 66, and a reflection bar 67 in addition to the objective lens 22, the dichroic mirror 46, and the perforated mirror 21.

Alignment light emitted from the LED 51 of the alignment optical system 50 travels through the diaphragms 52 and 53 and the relay lens 54 and is reflected by the dichroic mirror 55, passes through the aperture of the perforated mirror 21, penetrates the dichroic mirror 46, and is projected onto a cornea of the anterior eye part Ea of the subject eye E by the objective lens 22.

Cornea-reflected light of the alignment light travels through the objective lens 22, the dichroic mirror 46, and the aperture of the perforated mirror 21. After part of the cornea-reflected light penetrates the dichroic mirror 55, the part comes incident on the light-receiving surface of the image sensor 35 via the focal lens 31, the mirror 32, the half mirror 39A, the dichroic mirror 33, and the condenser lens 34.

The image sensor 35 picks up an image from the cornea-reflected light (receives the light) of the alignment light and outputs an image pickup signal. With this operation, an alignment indicator is displayed on the display device 3 together with the observation image described earlier of the anterior eye part Ea. An examiner performs alignment by the same operation as that for a conventional fundus camera. The calculation controlling unit 200 may perform alignment (auto-alignment) by analyzing a position of the alignment indicator and moving the optical system.

A reflection surface of the reflection bar 67 is set on an optical path of the illumination optical system 10 when focus adjustment by the focus optical system 60 is performed. Focus light emitted from the LED 61 passes through the relay lens 62 and is split into two pencils by the split indicator plate 63. After that, the focus light travels through the two-aperture diaphragm 64, the mirror 65, and the condenser lens 66 to form an image on the reflection surface of the reflection bar 67 and is reflected toward the relay lens 20 by the reflection surface. Further, the focus light is projected onto the fundus Ef via the relay lens 20, the perforated mirror 21, the dichroic mirror 46, and the objective lens 22.

Fundus-reflected light of the focus light passes through the same route as that of the cornea-reflected light of the alignment light and is subjected to image pickup by the image sensor 35. The image sensor 35 subjects the fundus-reflected light of the focus light to image pickup and outputs an image pickup signal. With this operation, the split indicator is displayed on the display device 3 together with the observation image. The calculation controlling unit 200 (to be described later) analyzes a position of the split indicator, moves the elements such as the focal lens 31, and automatically performs focusing in the same manner as before. The examiner may manually perform focusing while visually recognizing the split indicator.

The dichroic mirror 46 branches an optical path for OCT photographing from an optical path for fundus photographing. The dichroic mirror 46 reflects light in a wavelength range used for OCT photographing and transmits light for fundus photographing. A collimator lens unit 40, an optical path length changing unit 41, an optical scanner 42, a focal lens 43, a mirror 44, and a relay lens 45 are provided in the order from the OCT unit 100 side in the optical path for OCT measurement.

The optical path length changing unit 41 is movable in a direction indicated by an arrow shown in Figure 1 to change an optical path length of the measurement light LS (see Figure 2) for OCT photographing. The change in the optical path length is used for optical path length correction corresponding to an axial length of the subject eye E, interference state adjustment, and other purposes. The optical path length changing unit 41 includes, for example, a corner cube and a mechanism for moving the corner cube.

The optical scanner 42 is, for example, a galvanometer scanner and deflects (performs scanning with) the measurement light LS (see Figure 2) emitted from the OCT unit 100 (to be described later). The optical scanner 42 includes, for example, a galvanometer mirror for x-direction scanning with the measurement light LS, a galvanometer mirror for y-direction scanning with the measurement light LS, and a mechanism for driving the galvanometer mirrors independently. With this configuration, the measurement light LS can be used for scanning in an arbitrary direction on an xy plane, and the fundus Ef can be scanned in X and Y directions (subjected to two-dimensional scanning) with the measurement light LS.

The fundus camera unit 2 is provided with the one pair of anterior eye cameras 300. The one pair of anterior eye cameras 300 is a so-called stereo camera and substantially simultaneously photographs the anterior eye part Ea from different directions. The one pair of anterior eye cameras 300 is provided at a position off the optical path of the illumination optical system 10 and an optical path of the photographic optical system 30. The anterior eye cameras 300 acquire respective photographed images of the subject eye E in a state where the subject eye E is located at substantially the same position (oriented in substantially the same direction). The photographed images are used by the calculation controlling unit 200 to detect a relative position of the subject eye E to the fundus camera unit 2, and a result of the relative position detection is used for schematic alignment of the fundus camera unit 2 with respect to the subject eye E and other purposes.

Figure 2 is a schematic diagram of the optical system of the OCT unit 100. As shown in Figure 2 and Figure 1 described earlier, an OCT light source 101 of the OCT unit 100 is a light source of a wavelength swept type (wavelength scanning type) capable of sweeping (scanning) wavelengths of emitted light like a light source of a common OCT device of a swept source type and includes a laser light source including a resonator. The OCT light source 101 temporally changes an output wavelength in a near-infrared wavelength band that cannot be visually recognized by the human eye.

An optical system for executing swept source OCT is provided in the OCT unit 100. The optical system includes an interference optical system. The interference optical system has a function of splitting light L0 from the OCT light source 101 into the measurement light LS and reference light LR, a function of superimposing the return light LS1 of the measurement light LS from the subject eye E and the reference light LR after travel through a reference optical path to produce interference light LC, and a function of detecting the interference light LC. A detection result (detection signal) of the interference light LC obtained by the interference optical system is a signal indicating a spectrum of the interference light LC and is sent to the calculation controlling unit 200.

The light L0 output from the OCT light source 101 is guided by an optical fiber 102 to a polarization controller 103 and is adjusted in polarization state. The light L0 adjusted in polarization state is guided by an optical fiber 104 to a fiber coupler 105 and is split by the fiber coupler 105 into the measurement light LS and the reference light LR.

The reference light LR is guided by an optical fiber 110 to a collimator 111 to be converted into a parallel pencil and is guided to a corner cube 114 via an optical path length correcting member 112 and a dispersion compensation member 113. The optical path length correcting member 112 acts to match an optical path length of the reference light LR with that of the measurement light LS. The dispersion compensation member 113 acts to match dispersion characteristics of the reference light LR and the measurement light LS.

The corner cube 114 (retroreflector) and a corner cube moving mechanism 115 correspond to an optical path length changing unit of the present invention. The corner cube 114 is held by the corner cube moving mechanism 115 to be movable along an incident direction of the reference light LR. The corner cube moving mechanism 115 is an actuator that moves the corner cube 114 along the incident direction of the reference light LR, and the movement changes the optical path length of the reference light LR.

Note that although both the optical path length changing unit 41 that changes the optical path length of the measurement light LS and the corner cube 114 that changes the optical path length of the reference light LR are provided in the present embodiment, only either one of the optical path length changing unit 41 and the corner cube 114 may be provided. It is also possible to change an optical path length difference between the optical path length of the measurement light LS and that of the reference light LR using an optical member other than the optical path length changing unit 41 and the corner cube 114.

The reference light LR after the travel through the corner cube 114 travels through the dispersion compensation member 113 and the optical path length correcting member 112, is converted by a collimator 116 from a parallel pencil into a focused pencil, and comes incident on an optical fiber 117. The reference light LR incident on the optical fiber 117 is guided to a polarization controller 118 to be adjusted in polarization state, is guided by an optical fiber 119 to an attenuator 120 to be adjusted in light amount, and is guided by an optical fiber 121 to a fiber coupler 122.

The measurement light LS produced by the fiber coupler 105 is guided by an optical fiber 127 and is converted by the collimator lens unit 40 into a parallel pencil, and travels through the optical path length changing unit 41, the optical scanner 42, the focal lens 43, the mirror 44, and the relay lens 45. The measurement light LS after the travel through the relay lens 45 is reflected by the dichroic mirror 46, is refracted by the objective lens 22, and comes incident on the subject eye E. The measurement light LS is scattered and reflected at various depth positions of the subject eye E. The return light LS1 of the measurement light LS from the subject eye E advances reversely through the same route as that at the time of incidence and is guided to the fiber coupler 105, and reaches the fiber coupler 122 via an optical fiber 128.

The fiber coupler 122 produces the interference light LC between the return light LS1 incident via the optical fiber 128 and the reference light LR incident via the optical fiber 121. The fiber coupler 122 branches the interference light LC at a predetermined branching ratio (e.g., 1:1) to produce one pair of interference light components LC. The one pair of interference light components LC is guided to a detector 125 through optical fibers 123 and 124.

The detector 125 is, for example, a balanced photodiode. The balanced photodiode includes one pair of photodetectors that detect the one pair of interference light components LC and outputs a difference between one pair of detection results obtained from the photodetectors. The detector 125 sends the output (detection signal) to a DAQ 130 that is a data acquisition system.

The DAQ 130 is supplied with a clock KC from the OCT light source 101. The OCT light source 101 produces the clock KC in sync with the output timing of each wavelength of the light L0 to be swept within a predetermined wavelength range. The OCT light source 101, for example, produces the clock KC on the basis of a result of optically delaying one of two branch light components obtained by branching the light L0 of each output wavelength and then detecting combined light of the two branch light components. The DAQ 130 samples a detection signal input from the detector 125 on the basis of the clock KC.

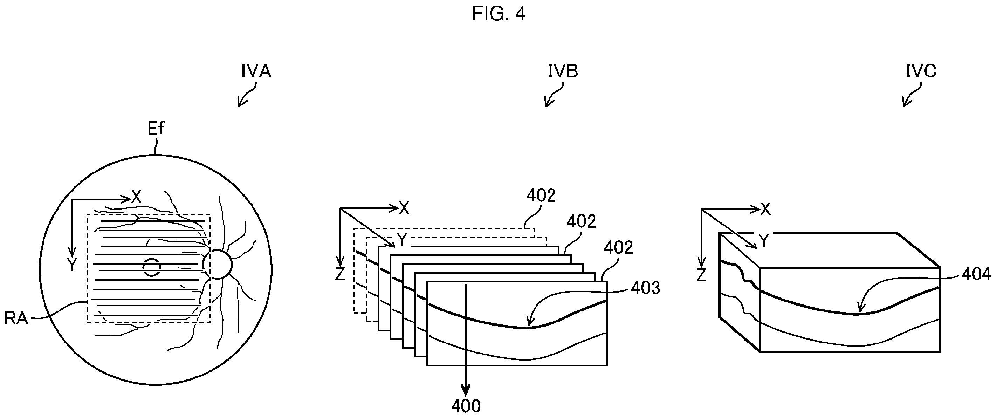

The DAQ 130 also sends a result of sampling a detection signal from the detector 125 to the calculation controlling unit 200. The calculation controlling unit 200 subjects a spectral distribution based on sampling data to calculation such as a Fourier transform for each of a series of wavelength scans (for each A-line) to form a reflection intensity profile for each A-line. The calculation controlling unit 200 further makes an image of the reflection intensity profile for each A-line and forms an A-scan image 400 to thereby form a B-scan image 402 of the fundus Ef, as shown in Figure 4 (to be described later).

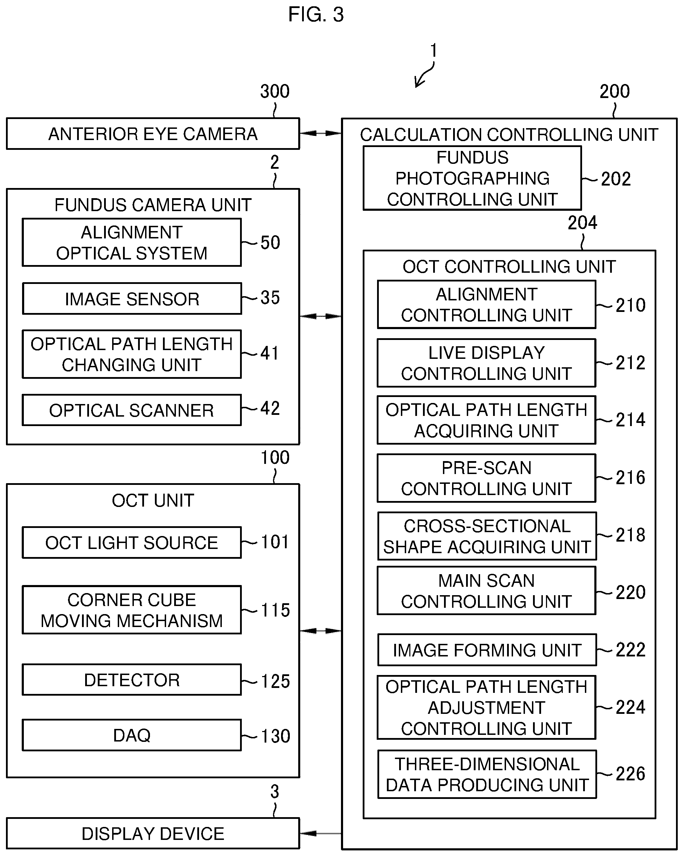

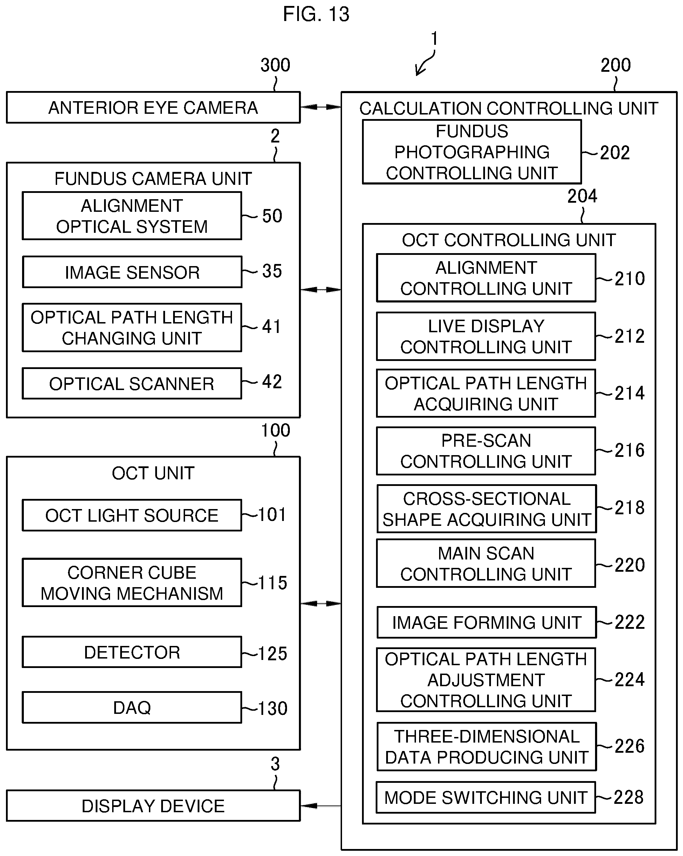

Figure 3 is a functional block diagram of the calculation controlling unit 200 according to the first embodiment. Note that components irrelevant to OCT photographing by the ophthalmic device 1 may not be shown in Figure 3 (the same applies to Figure 13 (to be described later)).

As shown in Figure 3, the calculation controlling unit 200 performs integrated control on operation of the units of the ophthalmic device 1. For example, an operation unit that accepts various types of input operations by the examiner, a movement mechanism (actuator) that moves the fundus camera unit 2 relative to the subject eye E (all not shown) are connected to the calculation controlling unit 200 in addition to the fundus camera unit 2, the display device 3, and the OCT unit 100.

The calculation controlling unit 200 includes a calculation circuit that includes various types of processors and memories. The various types of processors include a Central Processing Unit (CPU), a Graphics Processing Unit (GPU), an Application Specific Integrated Circuit (ASIC), and programmable logic devices (e.g., Simple Programmable Logic Devices (SPLDs), a Complex Programmable Logic Device (CPLD), and Field Programmable Gate Arrays (FPGAs)]. Note that various types of functions of the calculation controlling unit 200 may be implemented by one processor or may be implemented by processors of the same type or different types.

The calculation controlling unit 200 executes a control program that is stored in a storage unit (not shown), thereby functioning as a fundus photographing controlling unit 202 and an OCT controlling unit 204.

The fundus photographing controlling unit 202 controls the units of the fundus camera unit 2, the anterior eye cameras 300, the movement mechanism (not shown) and executes fundus photographing by the ophthalmic device 1. Note that specific processes of fundus photographing, such as alignment of the fundus camera unit 2 with respect to the subject eye E and fundus photographing by the fundus camera unit 2, are publicly known techniques and that a specific description of the processes will be omitted.

The OCT controlling unit 204 controls the units of the fundus camera unit 2, the units of the OCT unit 100, the anterior eye cameras 300, the movement mechanism (not shown) and executes alignment, OCT photographing of the fundus Ef by the ophthalmic device 1. The OCT photographing includes normal OCT photographing for acquiring a fundus tomographic image 403 that is a tomographic image of the fundus Ef and three-dimensional OCT photographing for acquiring three-dimensional data 404 (also referred to as a three-dimensional image, a three-dimensional model, or a C-scan image) of the fundus Ef, as shown in Figure 4 (to be described later). Note that the normal OCT photographing and functions of the OCT controlling unit 204 at the time of the normal OCT photographing are publicly known techniques and a specific description thereof will be omitted. A description will be given below on the assumption that the ophthalmic device 1 executes three-dimensional OCT photographing.

Figure 4 is an explanatory chart for explaining three-dimensional OCT photographing. As indicated by reference characters IVA and IVB in Figure 4, three-dimensional OCT photographing executes a 3D (H) scan (also referred to as a C-scan) that repeatedly executes a B-scan (line scan) on the fundus Ef in the X direction while changing a scanning position thereof in the Y direction, within a two-dimensional scanning range RA set in advance of the measurement light LS. With this operation, the B-scan image 402, in which the A-scan images 400 in the Z direction (A-scan direction) that is a depth direction of the fundus Ef are arrayed along the X direction (B-scan direction), is obtained for each scanning position of the 3D (H) scan.

The B-scan images 402 for the scanning positions of the 3D (H) scan include the respective fundus tomographic images 403. As a result, as indicated by reference character IVC in Figure 4, the three-dimensional data 404 of the fundus Ef can be produced on the basis of the B-scan images 402. In this case, the Z direction corresponds to an A-scan direction of the present invention, the X direction corresponds to a B-scan direction of the present invention, and a Y direction corresponds to a perpendicular direction of the present invention.

Note that the three-dimensional data 404 of the fundus Ef may be produced by executing a 3D (V) scan as a three-dimensional scan that repeatedly executes a B-scan in the Y direction on the fundus Ef while changing a scanning position thereof in the X direction, instead of executing a 3D (H) scan. In this case, the Y direction corresponds to the B-scan direction of the present invention, and the X direction corresponds to the perpendicular direction of the present invention.

Referring back to Figure 3, the OCT controlling unit 204 functions as an alignment controlling unit 210, a live display controlling unit 212, and an optical path length acquiring unit 214 before the start of three-dimensional OCT photographing. The OCT controlling unit 204 also functions as a pre-scan controlling unit 216, a cross-sectional shape acquiring unit 218, a main scan controlling unit 220, an image forming unit 222, an optical path length adjustment controlling unit 224, and a three-dimensional data producing unit 226 in executing three-dimensional OCT photographing.

The alignment controlling unit 210, for example, detects a relative position of the fundus camera unit 2 to the subject eye E on the basis of photographed images of the anterior eye part Ea photographed by the one pair of anterior eye cameras 300 and drives the movement mechanism (not shown) on the basis of a result of detecting the relative position, thereby executing schematic alignment of the fundus camera unit 2 with respect to the subject eye E. The alignment controlling unit 210 also executes projection of alignment light onto the anterior eye part Ea by the alignment optical system 50 and image pickup of cornea-reflected light of the alignment light by the image sensor 35. The alignment controlling unit 210 detects the relative position of the fundus camera unit 2 to the subject eye E on the basis of an image pickup signal output from the image sensor 35 and drives the movement mechanism (not shown) on the basis of a result of detecting the relative position, thereby executing precise alignment of the fundus camera unit 2 with respect to the subject eye E.

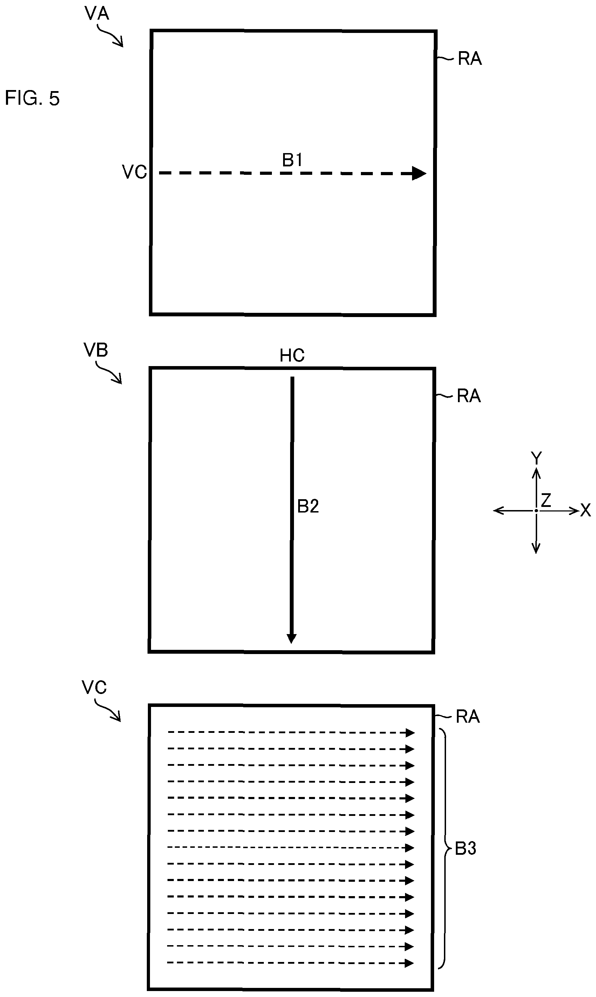

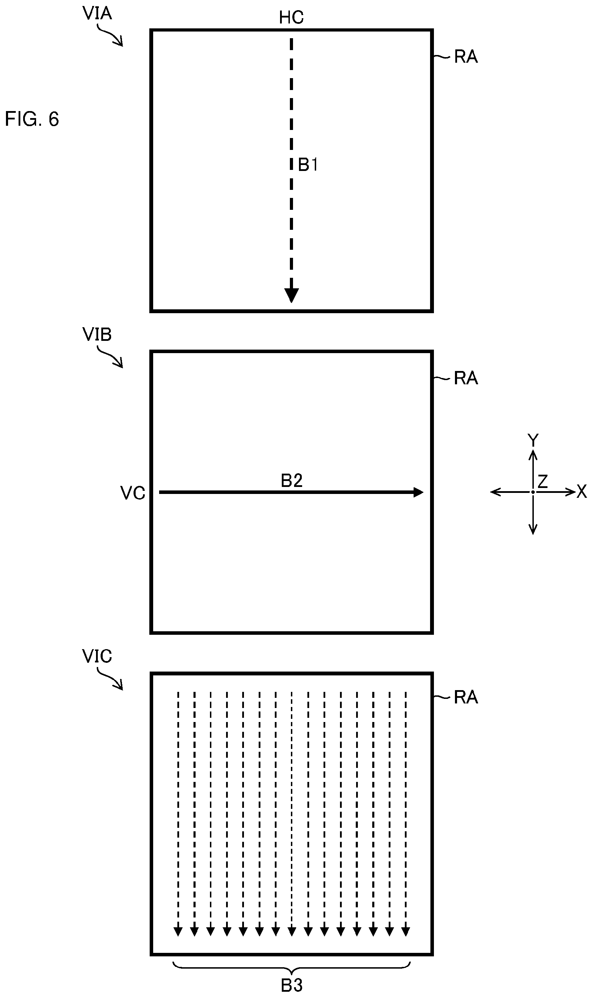

Figure 5 is an explanatory chart for explaining scanning with the measurement light LS from before three-dimensional OCT photographing [3D (H) scan] to completion of the photographing. Figure 6 is an explanatory chart for explaining scanning with the measurement light LS from before three-dimensional OCT photographing [3D (V) scan] to completion of the photographing.

As shown in Figure 3 and indicated by reference character VA in Figure 5, the live display controlling unit 212 controls the units of the fundus camera unit 2 and the units of the OCT unit 100 to execute OCT photographing for live display and display the fundus tomographic image 403 live on the display device 3, before three-dimensional OCT photographing [3D (H) scan].

Specifically, the live display controlling unit 212 controls the OCT light source 101 and the optical scanner 42 to repeatedly execute a B-scan in the X direction (see reference character B1) at a particular scanning position VC set in advance within the scanning range RA. The particular scanning position VC is set at, for example, an X-scan line passing through a central position in the Y direction of the scanning range RA. During the execution of the B-scans, the live display controlling unit 212 repeatedly executes detection of the interference light LC by the detector 125 and detection signal sampling by the DAQ 130. With this operation, the live display controlling unit 212 repeatedly executes formation of the B-scan image 402 of the fundus Ef and output of the B-scan image 402 to the display device 3 on the basis of sampling data output from the DAQ 130. This causes the B-scan image 402 of the fundus Ef to be displayed live on the display device 3.

As indicated by reference character VIA in Figure 6, the live display controlling unit 212 controls the OCT light source 101 and the optical scanner 42 to repeatedly execute a B-scan in the Y direction (see reference character B1) at a particular scanning position HC within the scanning range RA, before three-dimensional OCT photographing [3D (V) scan]. The particular scanning position HC is set at, for example, a Y-scan line passing through a central position in the X direction of the scanning range RA.

The live display controlling unit 212 adjusts an optical path length (hereinafter abbreviated as a target optical path length) of at least either one of the measurement light LS and the reference light LR during live display. Specifically, if a target optical path length that allows adjustment of a position of a predetermined region (desired region) of the fundus tomographic image 403 within the B-scan image 402 to a predetermined reference position (designated Z position) is an optimum optical path length, the live display controlling unit 212 executes optical path length adjustment that adjusts a target optical path length to the optimum optical path length on the basis of, e.g., a detection signal of the interference light LC detected by the detector 125 (see PTL 1 above). The reference position is appropriately set and adjusted such that the whole (almost the whole) of the fundus tomographic image 403 fits within the B-scan image 402 (frame) (see PTL 1 above).

The optical path length acquiring unit 214 acquires optimum optical path length information indicating the optimum optical path length adjusted by the live display controlling unit 212. The optimum optical path length information includes position information of, for example, the corner cube included in the optical path length changing unit 41 if a target optical path length includes the optical path length of the measurement light LS and includes position information of the corner cube 114 if the target optical path length includes the optical path length of the reference light LR. The optimum optical path length information also includes information indicating the particular scanning position VC or HC within the scanning range RA.

Note that timing of acquisition of the optimum optical path length information by the optical path length acquiring unit 214 is not particularly limited as long as the timing is at least before execution of a main scan (to be described later) [3D (H) scan or 3D (V) scan].

The pre-scan controlling unit 216, the cross-sectional shape acquiring unit 218, the main scan controlling unit 220, the image forming unit 222, the optical path length adjustment controlling unit 224, and the three-dimensional data producing unit 226 execute operation related to three-dimensional OCT photographing. Three-dimensional OCT photographing according to the present embodiment includes a pre-scan for grasping a cross-sectional shape of the fundus Ef and a main scan that executes the 3D (H) scan or the 3D (V) scan described earlier.

As shown in Figure 3 and indicated by reference character VB in Figure 5, the pre-scan controlling unit 216 controls the OCT light source 101 and the optical scanner 42 and executes a pre-scan (see reference character B2) that is a B-scan in the Y direction at the particular scanning position HC on the fundus Ef once (possibly a plurality of number of times) if a main scan is a 3D (H) scan. Note that a scanning position of the pre-scan may be shifted in the X direction from the particular scanning position HC.

The pre-scan controlling unit 216 also executes detection of the interference light LC by the detector 125 and detection signal sampling by the DAQ 130 during execution of the pre-scan.

Note that, as indicated by reference character VIB in Figure 6, the pre-scan controlling unit 216 controls the OCT light source 101 and the optical scanner 42 and executes a pre-scan (see reference character B2) that is a B-scan in the X direction at the particular scanning position VC on the fundus Ef once if the main scan is a 3D (V) scan. A scanning position of the pre-scan may be, for example, shifted in the Y direction from the particular scanning position VC.

As shown in Figure 3, the cross-sectional shape acquiring unit 218 forms the B-scan image 402 on the basis of sampling data output from the DAQ 130 in accordance with a detection signal of the interference light LC detected by the detector 125 during execution of a pre-scan. The cross-sectional shape acquiring unit 218 then detects the cross-sectional shape of the fundus Ef on the basis of the B-scan image 402.

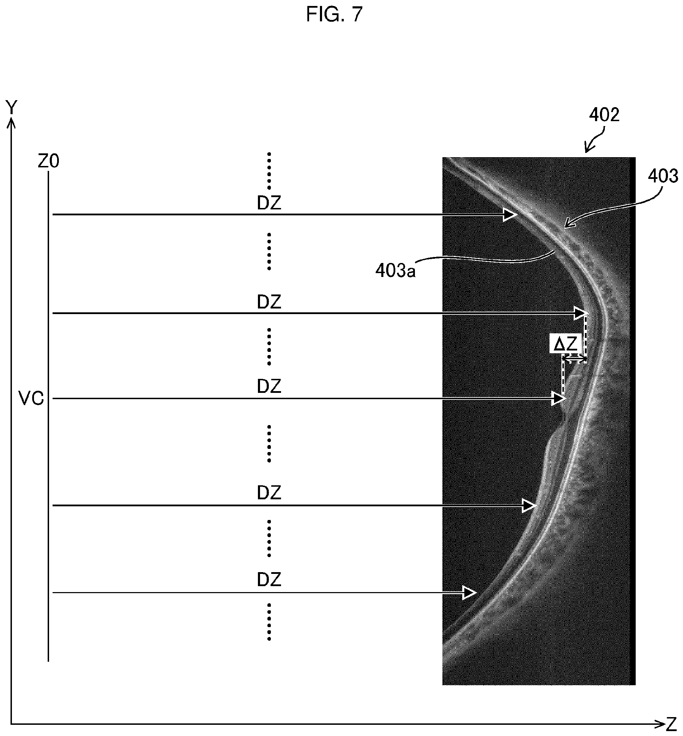

Specifically, the cross-sectional shape acquiring unit 218 detects a particular layer, such as an ILM layer 403a (see Figure 7) that is a layer of an internal limiting membrane (ILM), of the fundus Ef from the B-scan image 402 by a publicly known method. The cross-sectional shape acquiring unit 218 then detects the cross-sectional shape of the fundus Ef on the basis of a shape of the detected ILM layer 403a. This allows grasping of a change in the cross-sectional shape of the fundus Ef along a scanning direction of the pre-scan (see Figure 7).

The main scan controlling unit 220 controls the OCT light source 101 and the optical scanner 42 and executes, as a main scan, a 3D (H) scan indicated by reference character VC in Figure 5 or a 3D (V) scan indicated by reference character VIC in Figure 6 (see reference character B3), after acquisition of the cross-sectional shape of the fundus Ef by the cross-sectional shape acquiring unit 218. At this time, adjustment of a target optical path length by the optical path length adjustment controlling unit 224 (to be described later) is executed each time a scanning position of the 3D (H) scan is changed in the Y direction or each time a scanning position of the 3D (V) scan is changed in the X direction.

The main scan controlling unit 220 executes detection of the interference light LC by the detector 125 and detection signal sampling by the DAQ 130, during execution of the main scan.

The image forming unit 222 produces the B-scan image 402 for each scanning position of a main scan on the basis of sampling data output from the DAQ 130 in accordance with a detection signal of the interference light LC detected by the detector 125 during execution of the main scan.

Figure 7 is an explanatory chart for explaining adjustment of a target optical path length by the optical path length adjustment controlling unit 224 during execution of a main scan. Note that a description will be given in Figure 7, taking as an example a case where a 3D (H) scan is executed as the main scan.

As shown in Figures 3 and 7, the optical path length adjustment controlling unit 224 acquires optimum optical path length information from the optical path length acquiring unit 214 and acquires a cross-sectional shape along the Y direction of the fundus Ef from the cross-sectional shape acquiring unit 218. The optical path length adjustment controlling unit 224 drives at least one of the optical path length changing unit 41 and the corner cube moving mechanism 115 on the basis of the optimum optical path length information and the cross-sectional shape of the fundus Ef, thereby adjusting a target optical path length to an optimum optical path length for each scanning position of the 3D (H) scan. A description will be given below on the assumption that target optical path length adjustment is executed through movement of the corner cube 114 by the corner cube moving mechanism 115.

Specifically, the optical path length adjustment controlling unit 224 performs adjustment to an optimum optical path length adjusted by the live display controlling unit 212, i.e., an optimum optical path length indicated by the optimum optical path length information acquired by the optical path length acquiring unit 214 for the particular scanning position VC described earlier (including a scanning position near the particular scanning position VC) of scanning positions of the 3D (H) scan. With this operation, a position of the fundus tomographic image 403 within the B-scan image 402 obtained at the particular scanning position VC is adjusted to a reference position, and the whole of the fundus tomographic image 403 fits within the B-scan image 402.

The optical path length adjustment controlling unit 224 determines an optimum optical path length on the basis of the cross-sectional shape of the fundus Ef detected by the cross-sectional shape acquiring unit 218 and the optimum optical path length information acquired by the optical path length acquiring unit 214, for a scanning position other than the particular scanning position VC of the scanning positions of the 3D (H) scan.

Specifically, the optical path length adjustment controlling unit 224 detects a change in a Z position of the fundus Ef (the ILM layer 403a) along the Y direction on the basis of the cross-sectional shape of the fundus Ef detected by the cross-sectional shape acquiring unit 218. With this detection, the optical path length adjustment controlling unit 224 detects (estimates) a Z-direction distance DZ from an arbitrary Z reference (Z0) to the fundus Ef (the ILM layer 403a) for each scanning position of the 3D (H) scan. A difference in the Z-direction distance DZ between scanning directions indicates a difference in the optical path length of the measurement light LS. Thus, the optical path length adjustment controlling unit 224 calculates a difference ΔZ between the Z-direction distance DZ of the particular scanning position VC and the Z-direction distance DZ of a different scanning direction and corrects the optimum optical path length for the particular scanning position VC indicated by the optimum optical path length information on the basis of the difference ΔZ, thereby determining an optimum optical path length corresponding to the different scanning position.

As described above, the optical path length adjustment controlling unit 224 determines an optimum optical path length for each scanning position of the 3D (H) scan, thereby driving the corner cube moving mechanism 115 for each scanning position during execution of the 3D (H) scan and adjusting the target optical path length to the optimum optical path length. For this reason, even if the fundus Ef is greatly curved, it is possible to adjust the position (Z-direction position) of the fundus tomographic image 403 of the B-scan image 402 obtained for each scanning position to the reference position, i.e., fit the whole of the fundus tomographic image 403 within each B-scan image 402.

Note that, even if a 3D (V) scan is executed as the main scan, the optical path length adjustment controlling unit 224 determines an optimum optical path length for each scanning position of the 3D (V) scan, thereby driving the corner cube moving mechanism 115 for each scanning position during execution of the 3D (V) scan and adjusting the target optical path length to the optimum optical path length.

The three-dimensional data producing unit 226 produces the three-dimensional data 404 of the fundus Ef by a publicly known method on the basis of the B-scan image 402 for each scanning position of the main scan formed by the image forming unit 222. The three-dimensional data producing unit 226 outputs the produced three-dimensional data 404 to the display device 3 and the storage unit (not shown). With this operation, the three-dimensional data 404 is displayed by the display device 3 and is stored in the storage unit.

### [Operation of First Embodiment]

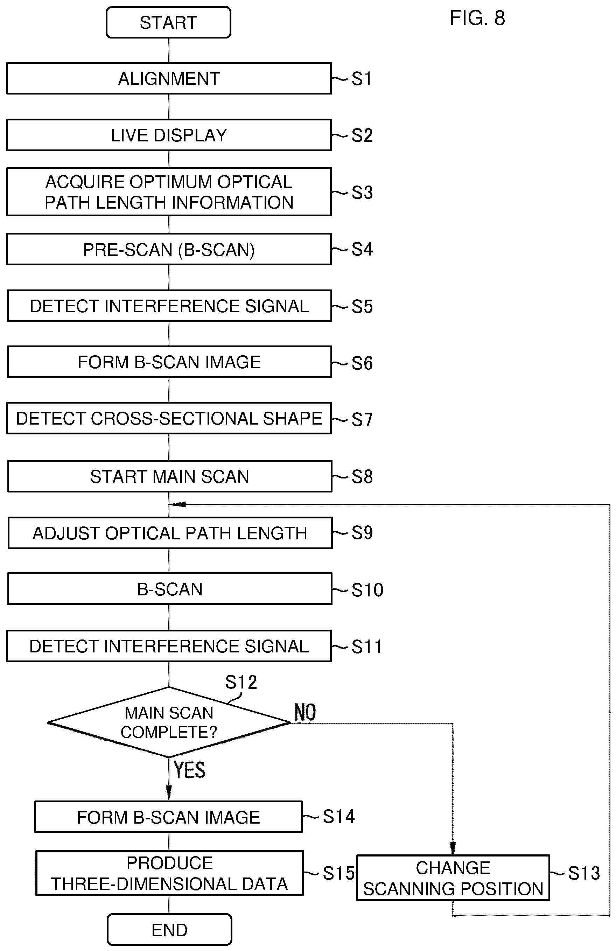

Figure 8 is a flowchart showing a flow of a three-dimensional OCT photographing process on the fundus Ef by the ophthalmic device 1 of the first embodiment, according to a control method for an ophthalmic device of the present invention. Note that a description will be given, taking as an example a case where a 3D (H) scan is executed as a main scan of three-dimensional OCT photographing.

As shown in Figure 8, after a face of a subject is supported by, e.g., a face support unit (not shown) of the ophthalmic device 1, the alignment controlling unit 210 uses the one pair of anterior eye cameras 300, the alignment optical system 50, the image sensor 35 to execute alignment (schematic alignment and precise alignment) of the fundus camera unit 2 with respect to the subject eye E (step S1). Note that publicly known focus adjustment is concurrently executed by a focus controlling unit (not shown).

After completion of the alignment, the live display controlling unit 212 controls the units of the fundus camera unit 2 and the units of the OCT unit 100 to execute OCT photographing for live display. The live display controlling unit 212 repeatedly executes formation of the B-scan image 402 at the particular scanning position VC and output of the B-scan image 402 to the display device 3. With this operation, the B-scan image 402 is displayed live on the display device 3 (step S2). At this time, the live display controlling unit 212 determines an optimum optical path length at the particular scanning position VC by a publicly known method, and drives the corner cube moving mechanism 115 and adjusts a target optical path length to the optimum optical path length.

The optical path length acquiring unit 214 acquires optimum optical path length information indicating the optimum optical path length at the particular scanning position VC adjusted by the live display controlling unit 212 before the start of a pre-scan and outputs the optimum optical path length information to the optical path length adjustment controlling unit 224 (step S3, corresponding to an optical path length acquiring step of the present invention).

Three-dimensional OCT photographing of the fundus Ef is started. First, the pre-scan controlling unit 216 controls the OCT light source 101 and the optical scanner 42 and executes a pre-scan (a B-scan in the Y direction) at the particular scanning position HC on the fundus Ef once (step S4), as indicated by reference character VB in Figure 5. Simultaneously, the pre-scan controlling unit 216 executes detection of the interference light LC by the detector 125 and detection signal sampling by the DAQ 130 during the execution of the pre-scan (step S5).

The cross-sectional shape acquiring unit 218 forms the B-scan image 402 on the basis of sampling data output from the DAQ 130 in accordance with a detection signal of the interference light LC detected by the detector 125 during the execution of the pre-scan (step S6). The cross-sectional shape acquiring unit 218 detects the cross-sectional shape of the fundus Ef by detecting the ILM layer 403a from the B-scan image 402 and outputs a result of detecting the cross-sectional shape to the optical path length adjustment controlling unit 224 (step S7, corresponding to a cross-sectional shape acquiring step of the present invention).

When the optical path length adjustment controlling unit 224 acquires the optimum optical path length information from the optical path length acquiring unit 214 and the cross-sectional shape detection result from the cross-sectional shape acquiring unit 218, the optical path length adjustment controlling unit 224 determines an optimum optical path length for each scanning position of the 3D (H) scan on the basis of the optimum optical path length information and the cross-sectional shape detection result, as shown in Figure 7. The main scan controlling unit 220 controls the OCT light source 101 and the optical scanner 42 to start a main scan [3D (H) scan] on the fundus Ef (step S8), as indicated by reference character VC in Figure 5.

First, the optical path length adjustment controlling unit 224 drives the corner cube moving mechanism 115 to adjust the target optical path length to an optimum optical path length corresponding to a first scanning position of the 3D (H) scan (step S9, corresponding to an optical path length adjustment controlling step). After the adjustment, the main scan controlling unit 220 controls the OCT light source 101 and the optical scanner 42 and executes a B-scan at the first scanning position (step S10). Simultaneously, the main scan controlling unit 220 executes detection of the interference light LC by the detector 125 and detection signal sampling by the DAQ 130 during the execution of the B-scan (step S11).

For a next scanning position of the 3D (H) scan (NO in step S12, and step S13), adjustment of the target optical path length to an optimum optical path length (step S9), a B-scan (step S10), and detection of the interference light LC and detection signal sampling (step S11) are executed. In the same manner as above, the process in step S13 and the processes in step S9 to step S11 are repeatedly executed for each of remaining scanning positions of the 3D (H) scan.

When the main scan [3D (H) scan] is completed (YES in step S12), the image forming unit 222 forms the B-scan image 402 for each scanning position of the main scan (step S14). Note that step S14 may be executed between step S11 and step S12.

The three-dimensional data producing unit 226 produces the three-dimensional data 404 of the fundus Ef by a publicly known method on the basis of the respective B-scan images 402 for the scanning positions of the main scan formed by the image forming unit 222 (step S15, corresponding to a three-dimensional data producing step of the present invention). The three-dimensional data 404 is displayed on the display device 3 and is stored in the storage unit (not shown).

### [Effects of First Embodiment]

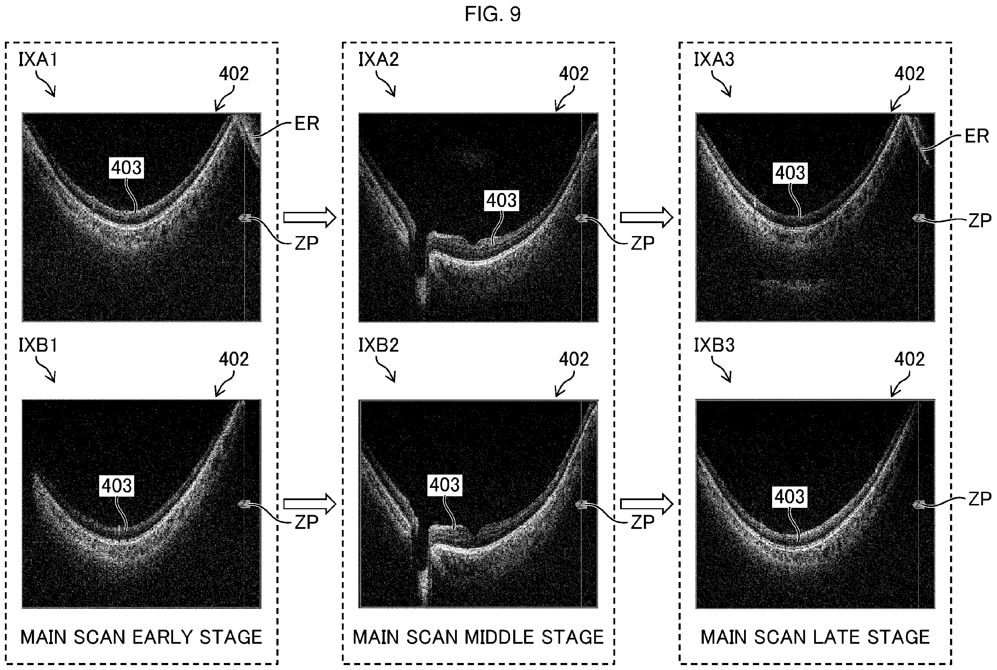

Figure 9 is an explanatory chart comparing B-scan images 402 (reference characters IXA1 to IXA3) at scanning positions of a main scan formed by a conventional method and B-scan images 402 (reference characters IXB1 to IXB3) at scanning positions of a main scan formed by the ophthalmic device 1 of the first embodiment. Note that reference character ZP in Figure 9 denotes a reference position for the fundus tomographic image 403 within the B-scan image 402.

As shown in Figure 9, while a scanning position in a main scan middle stage is close to the particular scanning position HC at the time of live display described earlier, a scanning position in a main scan early stage or a main scan late stage is away from the particular scanning position HC. For this reason, particularly when the scanning range RA is set wide, the difference ΔZ between the Z-direction distance DZ at a scanning position in the main scan early stage or the main scan late stage and the Z-direction distance DZ at the particular scanning position VC is large due to a curve of the fundus Ef, as shown in Figure 7.

If a main scan is executed in such a state by a conventional method, i.e., the main scan is executed with a set optimum optical path length corresponding to the particular scanning position VC, the fundus tomographic image 403 is adjusted to the reference position in the B-scan image 402 (see reference character IXA2) acquired at a scanning position in a main scan middle stage. In the B-scan image 402 (see reference characters IXA1 and IXA3) acquired at a scanning position in a main scan early stage or a main scan late stage, the fundus tomographic image 403 is not adjusted to the reference position, and folding (see reference character ER in Figure 9) may occur in a part of the fundus tomographic image 403.

In contrast, the ophthalmic device 1 of the first embodiment can adjust the fundus tomographic image 403 within the B-scan image 402 to the reference position at each scanning position by adjusting a target optical path length to an optimum optical path length for each scanning position of the main scan on the basis of the cross-sectional shape of the fundus Ef and optimum optical path length information at the particular scanning position HC (see reference characters IXB1 to IXB3). It is only required to determine an optimum optical path length for each scanning position of the main scan on the basis of the cross-sectional shape of the fundus Ef and the optimum optical path length information at the particular scanning position HC, and auto-Z need not be executed at each scanning position unlike before. As a result, the position of the fundus tomographic image 403 within the B-scan image 402 formed at each scanning position can be easily adjusted to the reference position. This allows easy production of the satisfactory three-dimensional data 404 of the fundus Ef.

### [Second Embodiment]

An ophthalmic device 1 according to a second embodiment of the present invention will be described. In the ophthalmic device 1 of the first embodiment, a pre-scan is executed only once, and the cross-sectional shape acquiring unit 218 detects a cross-sectional shape of the fundus Ef on the basis of a result of detecting the ILM layer 403a from the B-scan image 402 obtained by the pre-scan. Since the detection of the ILM layer 403a takes time, three-dimensional OCT photographing of the fundus Ef may require time. For this reason, the ophthalmic device 1 of the second embodiment executes three-dimensional OCT photographing of a fundus Ef in a shorter time than in the first embodiment.

Note that the ophthalmic device 1 of the second embodiment is basically the same in configuration as the ophthalmic device 1 of the first embodiment except that functions of a pre-scan controlling unit 216, a cross-sectional shape acquiring unit 218, and an optical path length adjustment controlling unit 224 are different from those in the first embodiment. For this reason, units identical in function or configuration to those in the first embodiment are denoted by identical reference numerals, and a description thereof will be omitted.

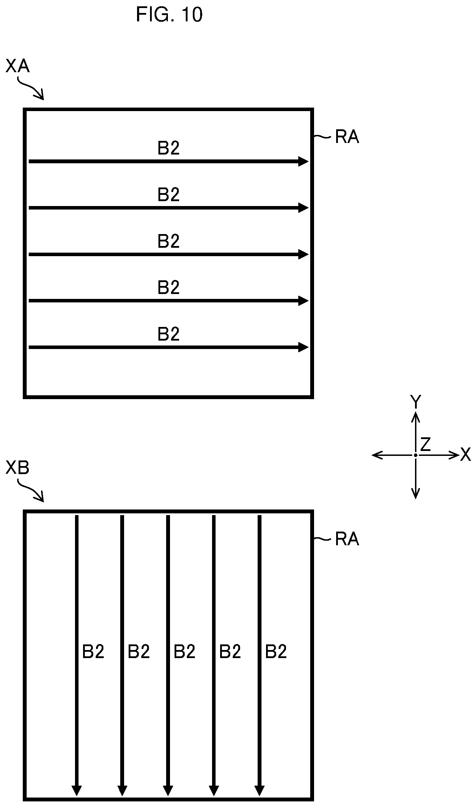

Figure 10 is an explanatory chart for explaining a pre-scan according to the second embodiment. Note that reference character XA in Figure 10 denotes a pre-scan to be executed before a 3D (H) scan and that reference character XB denotes a pre-scan to be executed before a 3D (V) scan.

As indicated by reference character XA in Figure 10, the pre-scan controlling unit 216 of the second embodiment controls an OCT light source 101 and an optical scanner 42 and executes B-scans (see reference character B2) on the fundus Ef in an X direction at a plurality of scanning positions different from each other in a Y direction if a main-scan is a 3D (H) scan. Note that the number of scanning positions of the pre-scan is not particularly limited.

As indicated by reference character XB in Figure 10, the pre-scan controlling unit 216 of the second embodiment controls the OCT light source 101 and the optical scanner 42 and executes B-scans (see reference character B2) on the fundus Ef in the Y direction at a plurality of scanning positions different from each other in the X direction if the main-scan is a 3D (V) scan.

The cross-sectional shape acquiring unit 218 of the second embodiment forms a B-scan image 402 for each scanning position of the pre-scan on the basis of sampling data that is output from a DAQ 130 in accordance with a detection signal of interference light LC detected by a detector 125 during the execution of the pre-scan. The cross-sectional shape acquiring unit 218 then predicts a cross-sectional shape of the fundus Ef on the basis of the B-scan images 402 at respective scanning positions.

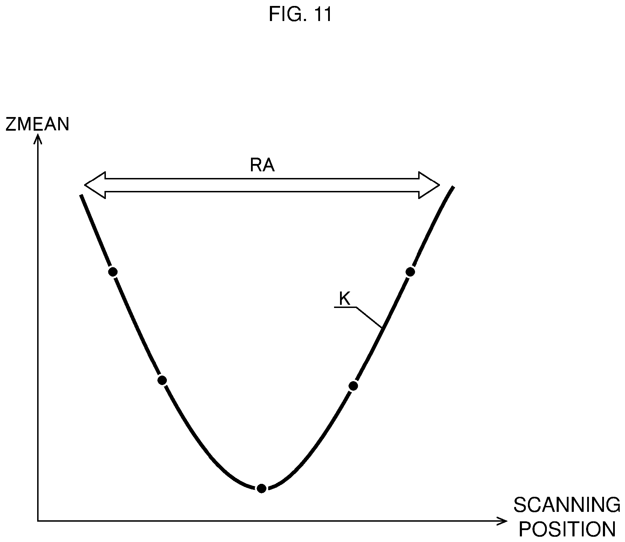

Figure 11 is an explanatory graph for explaining a process of predicting the cross-sectional shape of the fundus Ef by the cross-sectional shape acquiring unit 218 of the second embodiment. As shown in Figure 11, the cross-sectional shape acquiring unit 218 of the second embodiment calculates a Z position, such as ZMEAN representing an average height position in a Z direction, of a fundus tomographic image 403 within the B-scan image 402 from each of the B-scan images 402 at the respective scanning positions of the pre-scan by a publicly known method. The cross-sectional shape acquiring unit 218 of the second embodiment predicts the cross-sectional shape (see reference character K) of the fundus Ef on the basis of a result of calculating ZMEAN for each scanning position.

The optical path length adjustment controlling unit 224 of the second embodiment executes determination of an optimum optical path length for each scanning position of a main scan and adjustment of a target optical path length during the main scan on the basis of the cross-sectional shape of the fundus Ef predicted by the cross-sectional shape acquiring unit 218 and optimum optical path length information acquired by the optical path length acquiring unit 214, as in the first embodiment.

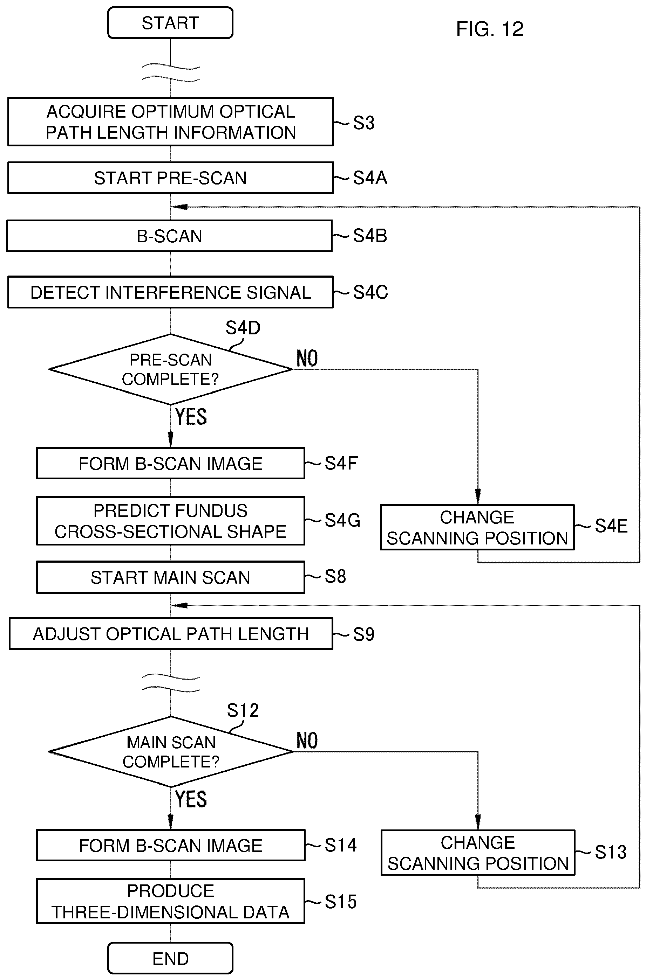

Figure 12 is a flowchart showing a flow of a three-dimensional OCT photographing process on the fundus Ef by the ophthalmic device 1 of the second embodiment. Note that processes up to step S3 are the same as those in the first embodiment shown in Figure 8 and that a description thereof will be omitted.

After the process in step S3, a pre-scan is started as in the first embodiment (step S4A). The pre-scan controlling unit 216 controls the OCT light source 101 and the optical scanner 42 and executes a B-scan at a first scanning position of the pre-scan (step S4B). Simultaneously, the pre-scan controlling unit 216 executes detection of the interference light LC by the detector 125 and detection signal sampling by the DAQ 130 during the execution of the B-scan (step S4C).

For a next scanning position of the pre-scan (NO in step S4D, and step S4E), a B-scan (step S4B), and detection of the interference light LC and detection signal sampling (step S4C) are executed. In the same manner, a process in step S4E and the processes in steps S4B and S4C are repeatedly executed for each of remaining B-scan scanning positions of the pre-scan.

When the pre-scan is completed (YES in step S4D), the cross-sectional shape acquiring unit 218 forms the B-scan image 402 for each scanning position of the pre-scan, and predicts the cross-sectional shape of the fundus Ef on the basis of a result of calculating ZMEAN from each B-scan image 402 (step S4G), as shown in Figure 11. As compared to a case of detecting the ILM layer 403a as in the first embodiment, the accuracy of the cross-sectional shape of the fundus Ef is lower, but the cross-sectional shape of the fundus Ef can be obtained in a shorter time.

Like the first embodiment shown in Figure 8, processes in step S8 and subsequent steps are executed.

As described above, the ophthalmic device 1 of the second embodiment predicts the cross-sectional shape of the fundus Ef on the basis of the B-scan images 402 obtained at a plurality of scanning positions of a pre-scan, thereby obtaining the cross-sectional shape of the fundus Ef in a short time. This results in execution of three-dimensional OCT photographing of the fundus Ef in a short time. Also, the same effects as those of the first embodiment can be obtained.

Note that although a B-scan in the X direction is executed at each scanning position of a pre-scan in the second embodiment if a main scan is a 3D (H) scan as shown in Figure 10, a B-scan scanning direction during the pre-scan is not particularly limited as long as the cross-sectional shape of the fundus Ef is predictable. Similarly, although a B-scan in the Y direction is executed at each scanning position of the pre-scan in the second embodiment if the main scan is a 3D (V) scan, a B-scan scanning direction during the pre-scan is not particularly limited.

### [Third Embodiment]

Figure 13 is a functional block diagram of a calculation controlling unit 200 of an ophthalmic device 1 according to a third embodiment. In each of the above-described embodiments, a pre-scan is executed before a main scan to detect or predict the cross-sectional shape of the fundus Ef. For example, if the scanning range RA is not wide, the position of the fundus tomographic image 403 within the B-scan image 402 formed at each scanning position of a main scan is generally close to a reference position even without execution of optical path length adjustment by the optical path length adjustment controlling unit 224. For this reason, if the scanning range RA is not wide, a pre-scan need not be executed. Skipping of a pre-scan allows completion of three-dimensional OCT photographing of the fundus Ef in a short time. Under the circumstances, the ophthalmic device 1 of the third embodiment automatically switches between execution and skipping of a pre-scan in accordance with a scanning range RA.

As shown in Figure 13, the ophthalmic device 1 of the third embodiment is basically the same in configuration as the ophthalmic devices 1 of the above-described embodiments except that the calculation controlling unit 200 further functions as a mode switching unit 228 and that functions of a pre-scan controlling unit 216, a cross-sectional shape acquiring unit 218, and an optical path length adjustment controlling unit 224 are partially different.

The mode switching unit 228 selectively switches an operation mode of a main scan to be executed by the ophthalmic device 1 (a main scan controlling unit 220) between normal mode and wide-angle mode in response to a mode switching operation input to an operation unit (not shown). The normal mode is a mode in which the scanning range RA is set at, for example, a range of 6 mm × 6 mm (corresponding to a first range of the present invention). The wide-angle mode is a mode in which the scanning range RA is set at a wider range than the normal mode, such as a range of 21 mm × 21 mm (corresponding to a second range of the present invention). Note that the scanning range RA for the normal mode and that for the wide-angle mode can be appropriately changed.

Note that, in the wide-angle mode, an examiner may attach a wide-angle lens (not shown) to an objective lens 22 or the wide-angle lens may be automatically attached by a lens insertion/removal mechanism (not shown) under control of the mode switching unit 228.

The pre-scan controlling unit 216, the cross-sectional shape acquiring unit 218, and the optical path length adjustment controlling unit 224 of the third embodiment are actuated only when the operation mode of the main scan is the wide-angle mode and are not actuated when the operation mode is the normal mode.

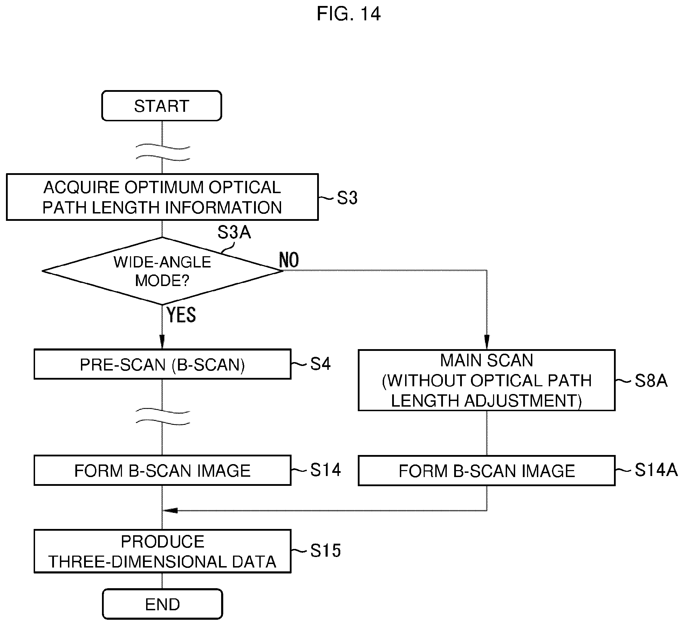

Figure 14 is a flowchart showing a flow of a three-dimensional OCT photographing process on the fundus Ef by the ophthalmic device 1 of the third embodiment. Note that processes up to step S3 are the same as in the first embodiment shown in Figure 8 and that a description thereof will be omitted.

If the mode switching unit 228 switches the operation mode of the main scan to the wide-angle mode in response to a mode switching operation input to the operation unit (not shown) (YES in step S3A), processes in step S4 and subsequent steps are executed in the same manner as in the first embodiment shown in Figure 8. Note that the processes in step S4A and the subsequent steps of the second embodiment shown in Figure 12 may be executed. This produces the same effects as those of the embodiments.

On the other hand, if the mode switching unit 228 switches the operation mode of the main scan to the normal mode (NO in step S3A), a pre-scan is not executed, and the main scan controlling unit 220 controls an OCT light source 101, an optical scanner 42, a detector 125, and a DAQ 130 to execute the main scan (step S8A). Optical path length adjustment by the optical path length adjustment controlling unit 224 is not executed during the execution of the main scan.

When the main scan is completed, an image forming unit 222 forms a B-scan image 402 for each scanning position of the main scan (step S14A). In the same manner as in the embodiments, a three-dimensional data producing unit 226 produces three-dimensional data 404 (step S15).

As described above, in the third embodiment, if the operation mode of the main scan is the normal mode, i.e., the scanning range RA is not wide, three-dimensional OCT photographing of the fundus Ef can be completed in a short time by skipping optical path length adjustment during the pre-scan and the main scan.

### [Others]

Although optical path length adjustment by the optical path length adjustment controlling unit 224 is executed for each scanning position of a main scan in the above-described embodiments, optical path length adjustment may be executed, for example, each time a scanning position advances by a fixed number of lines, i.e., intermittently. Optical path length adjustment may be executed only at a scanning position where the difference ΔZ exceeds a threshold. To cope with strong curves at two ends of a cross-section of the fundus Ef, as in the cross-sectional shape of the fundus Ef shown in Figure 7, the optical path length adjustment controlling unit 224 may execute optical path length adjustment at each scanning position or at short intervals in a main scan early stage and a main scan late stage and execute optical path length adjustment at long intervals in a main scan middle stage.

Although the ophthalmic device 1 executes three-dimensional OCT photographing of the fundus Ef of the subject eye E in the embodiments, the present invention is also applicable to a case of executing three-dimensional OCT photographing of another site to be observed of the subject eye E.

Although a B-scan scanning position is changed in a direction perpendicular to both an A-scan direction and a B-scan direction during execution of a main scan in the embodiments, a method for changing a B-scan scanning position is not particularly limited as long as a whole surface of the scanning range RA can be scanned.

Although a description has been given in the embodiments, taking as an example the ophthalmic device 1 that performs OCT photographing of a swept source type, the present invention is applicable to, for example, the ophthalmic device 1 that executes OCT photographing of various publicly known types, such as OCT photographing of a spectral domain type.

Although a description has been given in the embodiments, taking as an example of the ophthalmic device 1 a multifunction machine that is a combination of a fundus camera and an optical coherence tomograph, the ophthalmic device 1 may be composed only of an optical coherence tomograph. The present invention is applicable to various types of devices capable of executing three-dimensional OCT photographing of a site to be observed of the subject eye E.

### {Reference Signs List}

1 ophthalmic device
2 fundus camera unit
3 display device
10 illumination optical system
11 observation light source
12 reflection mirror
13 condenser lens
14 visible light cut filter
15 photographing light source
16 mirror
17 relay lens
18 relay lens
19 diaphragm
20 relay lens
21 perforated mirror
22 objective lens
30 photographic optical system
31 focal lens
32 mirror
33 dichroic mirror
34 condenser lens
35 image sensor
36 mirror
37 condenser lens
38 image sensor
39 target displaying unit
39A half mirror
40 collimator lens unit
41 optical path length changing unit
42 optical scanner
43 focal lens
44 mirror
45 relay lens
46 dichroic mirror
50 alignment optical system
51 LED
52 diaphragm
53 diaphragm
54 relay lens
55 dichroic mirror
60 focus optical system
61 LED
62 relay lens
63 split indicator plate
64 two-aperture diaphragm
65 mirror
66 condenser lens
67 reflection bar
90 fixation target
100 OCT unit
101 OCT light source
102 optical fiber
103 polarization controller
104 optical fiber
105 fiber coupler
110 optical fiber
111 collimator
112 optical path length correcting member
113 dispersion compensation member
114 corner cube
115 corner cube moving mechanism
116 collimator
117 optical fiber
118 polarization controller
119 optical fiber
120 attenuator
121 optical fiber
122 fiber coupler
123 optical fiber
124 optical fiber
125 detector
127 optical fiber
128 optical fiber
200 calculation controlling unit
202 fundus photographing controlling unit
204 OCT controlling unit
210 alignment controlling unit
212 live display controlling unit
214 optical path length acquiring unit
216 pre-scan controlling unit
218 cross-sectional shape acquiring unit
220 main scan controlling unit
222 image forming unit
224 optical path length adjustment controlling unit
226 three-dimensional data producing unit
228 mode switching unit
300 anterior eye camera
400 A-scan image
402 B-scan image
403 fundus tomographic image
403a ILM layer
404 three-dimensional data
DZ Z-direction distance
E subject eye
Ea anterior eye part
Ef fundus
HC particular scanning position
KC clock
L0 light
LC interference light
LR reference light
LS measurement light
LS1 return light
RA scanning range
VC particular scanning position
ΔZ difference

## Claims

1. An ophthalmic device comprising:
an interference optical system configured to split light emitted from a light source into measurement light and reference light, irradiate a site to be observed of a subject eye with the measurement light, and detect interference light between the reference light and return light from the site to be observed irradiated with the measurement light;
an optical path length changing unit provided in the interference optical system and configured to change an optical path length of at least one of the measurement light and the reference light;
an optical scanner configured to scan the site to be observed with the measurement light;
a pre-scan controlling unit configured to control the optical scanner and execute a pre-scan that is a B-scan on the site to be observed;
a cross-sectional shape acquiring unit configured to form a B-scan image of the site to be observed on the basis of a detection signal of the interference light detected by the interference optical system during execution of the pre-scan and detect or predict a cross-sectional shape of the site to be observed on the basis of the B-scan image;
a main scan controlling unit configured to control the optical scanner and execute, as a main scan, a three-dimensional scan that repeatedly executes the B-scan while changing a scanning position of the B-scan on the site to be observed;
an optical path length acquiring unit configured to, if the optical path length that allows adjustment of a position of a tomographic image of the site to be observed within the B-scan image to a reference position set in advance is an optimum optical path length, acquire the optimum optical path length at a particular scanning position of the main scan at least before execution of the main scan;
an optical path length adjustment controlling unit configured to, during execution of the main scan, control the optical path length changing unit to adjust the optical path length to the optimum optical path length on the basis of the cross-sectional shape detected or predicted by the cross-sectional shape acquiring unit and the optimum optical path length acquired by the optical path length acquiring unit; and
a three-dimensional data producing unit configured to produce three-dimensional data of the site to be observed on the basis of the detection signal of the interference light detected by the interference optical system during execution of the main scan.

2. The ophthalmic device according to claim 1, wherein if a direction in which the B-scan on the site to be observed is to be performed, the direction being perpendicular to both an A-scan direction and a B-scan direction, is a perpendicular direction, the main scan controlling unit controls the optical scanner, and repeatedly executes, as the main scan, the B-scan while changing the scanning position along the perpendicular direction.

3. The ophthalmic device according to claim 2, wherein
the pre-scan controlling unit controls the optical scanner and executes, as the pre-scan, the B-scan in the perpendicular direction on the site to be observed, and
the cross-sectional shape acquiring unit detects a particular layer of the site to be observed from the B-scan image and detects the cross-sectional shape on the basis of a shape of the particular layer.

4. The ophthalmic device according to claim 1, wherein
the pre-scan controlling unit controls the optical scanner and executes, as the pre-scan, the B-scan at a plurality of the scanning positions different from each other, and
the cross-sectional shape acquiring unit forms a plurality of the B-scan images corresponding one-to-one with the scanning positions of the pre-scan and predicts the cross-sectional shape on the basis of a result of detecting the position of the tomographic image within the B-scan image for each of the scanning positions.

5. The ophthalmic device according to any one of claims 1 to 4, wherein
an operation mode of the main scan by the main scan controlling unit is selectively switchable between normal mode in which a scanning range of the measurement light is set at a first range and wide-angle mode in which the scanning range is set at a second range wider than the first range, and
the pre-scan controlling unit, the cross-sectional shape acquiring unit, the optical path length acquiring unit, and the optical path length adjustment controlling unit are actuated only when the operation mode is the wide-angle mode.

6. The ophthalmic device according to any one of claims 1 to 4, comprising:
a display device; and
a live display controlling unit configured to execute, before the pre-scan, the B-scan at the particular scanning position by the optical scanner, and formation of the B-scan image based on the detection signal of the interference light detected by the interference optical system and display of the B-scan image by the display device, wherein
the live display controlling unit adjusts the optical path length to the optimum optical path length on the basis of the detection signal of the interference light detected by the interference optical system, and
the optical path length acquiring unit acquires the optimum optical path length adjusted by the live display controlling unit before the pre-scan.

7. The ophthalmic device according to any one of claims 1 to 4, comprising
an image forming unit configured to form the respective B-scan images for the scanning positions on the basis of respective detection signals of the interference light detected by the interference optical system at the scanning positions during execution of the main scan, wherein
the three-dimensional data producing unit comprises a three-dimensional data producing unit configured to produce the three-dimensional data on the basis of the respective B-scan images formed for the scanning positions by the image forming unit.

8. A control method for an ophthalmic device including:
an interference optical system configured to split light emitted from a light source into measurement light and reference light, irradiate a site to be observed of a subject eye with the measurement light, and detect interference light between the reference light and return light from the site to be observed irradiated with the measurement light,
an optical path length changing unit provided in the interference optical system and configured to change an optical path length of at least one of the measurement light and the reference light, and
an optical scanner configured to scan the site to be observed with the measurement light, the method comprising:
a cross-sectional shape acquiring step of forming a B-scan image of the site to be observed on the basis of a detection signal of the interference light detected by the interference optical system during execution of a pre-scan that is a B-scan on the site to be observed by the optical scanner and detecting or predicting a cross-sectional shape of the site to be observed on the basis of the B-scan image;
an optical path length acquiring step of, if a three-dimensional scan that repeatedly executes the B-scan while changing a scanning position of the B-scan on the site to be observed by the optical scanner is a main scan, and the optical path length that allows adjustment of a position of a tomographic image of the site to be observed within the B-scan image to a reference position set in advance is an optimum optical path length, acquiring the optimum optical path length at a particular scanning position of the main scan at least before execution of the main scan;
an optical path length adjustment controlling step of, during execution of the main scan, controlling the optical path length changing unit to adjust the optical path length to the optimum optical path length on the basis of the cross-sectional shape detected or predicted in the cross-sectional shape acquiring step and the optimum optical path length acquired in the optical path length acquiring step; and
a three-dimensional data producing step of producing three-dimensional data of the site to be observed on the basis of the detection signal of the interference light detected by the interference optical system during execution of the main scan.
